# EUROPÄISCHE PATENTSCHRIFT

(11) **EP 2 702 950 B1**
(45) Veröffentlichungstag und Bekanntmachung des Hinweises auf die Patenterteilung: **20.05.2015**
(21) Anmeldenummer: 12182283.7
(22) Anmeldetag: 30.08.2012
(51) Int. Cl.: A61B 17/16, A61C 1/10, B23B 31/00, B23B 31/107, A61B 17/00

(54) **Medizinisches Werkzeugsystem, sowie Handgriff und Werkzeug für ein medizinisches Werkzeugsystem**
Medical tool system and handle and tool for a medical tool system
Système d'outil médical, ainsi que poignée et outil pour un système d'outil médical

(43) Veröffentlichungstag der Anmeldung: 05.03.2014
(73) Patentinhaber: Rotomed AG, 4512 Bellach (CH)
(72) Erfinder: Müller, Thomas, 4513 Langendorf (CH); Müller, Daniel, 4513 Langendorf (CH)
(74) Vertreter: Patentbüro Paul Rosenich AG

(56) Entgegenhaltungen:
- EP-A1- 1 563 801
- EP-A2- 1 386 587
- WO-A1-97/00149
- US-A- 5 624 214
- US-A- 5 888 200
- US-A1- 2006 041 268
- US-B2- 6 562 055

## Beschreibung

Die Erfindung betrifft einen Handgriff für ein medizinisches Werkzeugsystem, mit einem Gehäuse mit einem Rotationsantrieb für ein medizinisches Werkzeug an einer axial fixierten Position Innerhalb des Gehäuses, sowie einer vom Handgriff distal ausgehenden, axial fixierten Führungshülse für das Werkzeug und mit einem manuell betätigbaren, einstellbaren Teleskoping-Mechanismus für das Werkzeug, nach dem Oberbegriff des Anspruchs 1. Des Weiteren betrifft die Erfindung ein medizinisches Werkzeugsystem nach dem Oberbegriff des Anspruchs 7.

In der Chirurgie aber auch in der Dentalmedizin werden vielfach Werkzeugsysteme eingesetzt, bei welchen ein Schneide-, Bohr- oder ähnliches Werkzeug in einem Handgriff gehalten und von einem Rotationsantrieb, beispielsweise einem Druckluftantrieb, in Drehung versetzt werden. Dabei besteht oftmals die Notwendigkeit, dass die Werkzeugspitze gegenüber dem Handgriff bzw. gegenüber der werkzeugführenden Hand des Chirurgen oder Zahnarztes unterschiedlich weit entfernt ist und vorzugsweise durch einfache Manipulation am Handgriff axial verschoben werden kann. Es sind verschiedene Systeme bekannt, wie dies bewerkstelligt werden kann.

Für Handwerker-Werkzeuge ist in der US 5624214 A ein teleskopartig längsverstellbares Verlängerungsstück für Bohrwerkzeuge offenbart. Es beruht auf dem Prinzip, dass ein im Bohrfutter fixierter Stab mit mehreren in Längsrichtung aufeinanderfolgenden Ausnehmungen versehen ist, in welche Haltekugeln eingedrückt werden können, die in einer auf dem Stab längsverschieblichen Hülse gehalten sind. Die Kugeln können durch eine axial gegenüber der ersten Hülse verschiebbare weitere Hülse mit innerer Schulter in die Ausnehmungen des Stabes gedrückt oder daraus freigegeben werden. Auch die Fixierung des eigentlichen Bohrwerkzeuges in der Verlängerung wird durch das gleiche Prinzip bewerkstelligt. Einen Führungshülse für das Werkzeug ist nicht vorhanden.

Auf medizintechnischem Gebiet offenbart die EP 837749 B1 ein Werkzeugsystem, bei welchem der unterschiedliche Abstand zwischen Werkzeugspitze und Handgriff durch Ausstattung des Basishandgriffs mit unterschiedlich langen distalen Hülsen erzeugt wird. Durch eine in dieser Hülse gelagerte Verbindungswelle wird der Abstand zwischen dem Rotationsantrieb und dem proximalen Ende des in Hülse eingesetzten Werkzeugs überbrückt, so dass mit Werkzeugen einheitlicher Länge dennoch unterschiedliche Abstände zwischen Werkzeugspitze und werkzeugführender Hand erzielt werden können. Hülse und Verbindungswelle werden über Schnellkupplungen mit dem Handgriff bzw. dem Rotationsantrieb verbunden, ebenso wie das Werkzeug mit der Verbindungswelle oder allenfalls direkt dem Rotationsantrieb. Für den Umbau sind jedoch eigene Werkzeuge erforderlich, und jede vorhandene Kupplung ist eine Quelle von Störungen, unerwünschten Vibrationen und erfordert zusätzlichen baulichen Aufwand. Auch ist die Manipulation mit vergleichsweise kleinen Bauteilen, wie den erwähnten Hülsen und Verbindungswellen - vor allem, wenn man diese Manipulation unter Operationsstress und mit Operationshandschuhen durchzuführen hat, problematisch und zeitaufwendig, da für einen gewünschten Wechsel vier Wechselvorgänge vorzunehmen sind:
1) Werkzeug raus
2) Hülse weg
3) Neue Hülse rein
4) Werkzeug rein

Die US 6562055 B2 hingegen beschreibt für einen einzelnen Handgriff eine Möglichkeit zur Abstandseinstellung zwischen distaler Werkzeugspitze und Handgriff des Werkzeugsystems durch axiale Verstellbarkeit des eingesetzten Werkzeugs gegenüber dem Handgriff innerhalb einer fixen, längenkonstanten Führungshülse. Dazu ist das Werkzeug am proximalen Ende, an dem es mit dem Rotationsantrieb gekoppelt und in einem Aufnahmemechanismus im Handgriff verriegel werden kann, mit über einen bestimmten Längsbereich verteilten Einkerbungen versehen, in welche Haltelaschen des Aufnahmemechanismus eingreifen können. Diese Haltelaschen sind aussen mit einer schrägen Schulter versehen und werden durch innere schräge Schultern an einer federbeaufschlagten, die Haltelaschen umgebenden Manschette radial in die Einkerbungen des Aufsatzes eingreifende Stellung gedrückt. Dieser Aufbau wird in der Fachsprache als Teleskoping bezeichnet und hat sich in bestimmten Bereichen als Standard durchgesetzt. Für das Teleskoping ist per erfindungsgemäss verwendeter Definition ein Mechanismus vorgesehen, der den Schaft eines im Handgriff eingesetzten Werkzeugs axial verschiebbar und arretierbar macht.

Beim Teleskoping spricht man von einer kurzen und einer langen Position (des Werkzeugs). Die kurze Position ist jene, bei der das distale Ende des Werkzeugs dem Handgriff nah ist, während bei der langen Position das distale Ende des Werkzeugs dem Handgriff fern ist.

Vorteil dieser bekannten Variante ist, dass anstelle von vier Wechselvorgängen nur mehr ein einziger Wechselvorgang stattfindet.

### 1) Betätigung des Teleskoping-Mechanismus auf die gewünschte Länge.

Nachteilig an diesem System ist aber, dass mit einem Werkzeug nur eine beschränkte Längenverstellung möglich ist, und für einen grösseren Verstellbereich unterschiedlich lange Werkzeuge benötigt werden, was erhöhten Aufwand in der Herstellung und Lagerhaltung sowie der Handhabung der Werkzeuge hervorruft. Es sind dabei nicht nur Werkzeuge mit unterschiedlicher Ausführung der Spitze sondern auch für jede Spitzenausführung mit unterschiedlicher Länge erforderlich. Dies erhöht den Wechselvorgang wieder auf zwei bis drei:
1) Werkzeug raus
2) Neues Werkzeug rein
3) Betätigung des Teleskoping-Mechanismus auf die gewünschte Länge des neuen Werkzeugs.

Ausserdem entsteht bei die Führungshülse weit überragenden Werkzeugen ein langer, radial ungeführter Bereich des Werkzeugs bzw. Werkzeug-Schafts, was zu erhöhtem Verschleiss beispielsweise In den Lagern, aber auch zu einer erhöhten Patientenbelastung durch den rotierenden Werkzeugschaft im Gewebebereich führen kann.

Eine naheliegende bekannte Variante ist des Weiteren das Verwenden eines Handgriffs mit - wie oben angegebenen - einem Satz von identischen Werkzeugen mit verschieden langen Werkzeugschäften, ohne Teleskoping-Mechanismus.

Diese Variante verliert die Eigenschaft, einen raschen Wechsel der Eingriffstiefe (durch das Teleskoping) zu ermöglichen und benötigt jeweils wenigstens zwei Wechselvorgänge:
1) Werkzeug raus
2) Neues Werkzeug rein.

Aufgabe der vorliegenden Erfindung ist die neuartige Gestaltung eines Handgriffes für ein medizinisches Werkzeugsystem, um bei einfacher Bauweise und mit geringer Anzahl an Komponenten eine möglichst grosse Abstandsverstellung zwischen Werkzeugspitze und werkzeugführender Hand bei gleichzeitiger Vereinfachung der lösbaren Verbindung zwischen eingesetztem Werkzeug und Handgriff zu erzielen.

Eine weitere Aufgabe der Erfindung ist es somit, ein Werkzeugsystem derart optimiert auszulegen, dass bei einfacher Bauweise und mit geringer Anzahl an Komponenten eine möglichst grosse Abstandsverstellung zwischen Werkzeugspitze und werkzeugführender Hand zu erzielen ist, ohne aber Probleme betreffend seitlicher Führung des Werkzeugs oder erhöhter Patientenbelastung hervorzurufen.

Die erste Aufgabe wird durch die Merkmale des unabhängigen Anspruchs 1 und die zweite Aufgabe wird durch die Merkmale des unabhängigen Anspruchs 7 gelöst. Vorteilhafte Weiterbildungen sind in den Figuren, deren Beschreibung und in den abhängigen Patentansprüchen dargelegt.

Gemäss einem ersten Aspekt der Erfindung Ist ein Handgriff für ein medizinisches Werkzeugsystem vorgesehen, bei welchem der manuell betätigbare Einstellmechanismus durch eine Mehrzahl von axial beabstandeten Einherbungen im proximalen Ende der Verlängerungswelle, durch zumindest eine im Gehäuse axial fixiert aber radial beweglich gehaltene Haltekugel und eine axial durch eine Feder beaufschlagte, die Haltekugeln umfassende und manuell betätigbare Schliesshülse gebildet Ist, deren innerer Durchmesser sich in axialer Richtung ändert, wobei ein kleinerer Durchmesser die oder jede Haltekugel in jeweils eine Einkerbung der Verlängerungswelle drückt und ein grösserer Durchmesser die oder jede Haltekugel aus der jeweiligen Einkerbung freigibt.

Dabei liegt erfindungsgemäss die am weitesten proximale Einkerbung an der Verlängerungswelle radial weiter aussen als alle sich distal anschliessenden Einkerbungen. Durch dieses Merkmal ist eine weitere axiale Position des Einstellmechanismus gegeben, die nur bei von der normalen Längenverstellung abweichender manueller Betätigung eingestellt werden kann. Diese Position kann für eine von der normalen Längenverstellung abweichende Funktion genutzt werden, beispielsweise für die Entriegelung des Werkzeugs bzw. die Verstellung des Aufnahmemechanismus in die für die Aufnahme des Werkzeugs vorgesehene Position und Stellung.

Derart kann sichergestellt werden, dass beim Wechsel des Handgriffs die jeweilige Aufnahmeposition immer die gleiche (lange) Position ist. Dies erleichtert dem Chirurgen, die Orientierung mit dem Werkzeug und seinem Teleskoping-Mechanismus. Die Wechselvorgänge sind dann wie folgt:
1) Betätigung des Teleskoping-Mechanismus auf die kurze Position des Werkzeugs
2) Handgriff weg
3) Neuer Handgriff ran... Dieser hat dann automatisch die lange Position des Werkzeugs eingestellt.

Durch die Anordnung des Verstellmechanismus zwischen Rotationsantrieb und Verlängerungswelle können das proximale Ende des Werkzeugs und der Aufnahmemechanismus optimal und ausschliesslich auf die Halte- und Verriegelungsfunktion ausgelegt und somit baulich vereinfacht werden, was auch die Sicherheit der Aufnahme und Verriegelung erhöht.

Der oben beschriebene Handgriff ist sowohl als unabhängige neuartiger - Teleskoping - Bauteil verwendbar, da er eine besonders vorteilhafte Ausführungsform des Telescoping-Verstellmechanismus beinhaltet, als auch als Systemkomponente für ein medizinisches Werkzeugsystem gemäss einem der vorhergehenden Ansprüche, mit der jeweiligen erforderlichen Länge der Führungshülse, und zur Verwendung mit einem Werkzeug aus einem Set von Werkzeugen mit identischer Länge des Werkzeug-Schafts.

Gemäss einer bevorzugten Ausführungsform der Erfindung ist dabei vorgesehen, dass die mit der oder jeder Haltekugel zusammenwirkende Innenwandung der Schliesshülse zumindest im Bereich des grösseren inneren Durchmessers konisch geformt ist, wobei der Konuswinkel im Bereich des grösseren inneren Durchmessers grösser ist als im Bereich des kleineren inneren Durchmessers. Ohne die Kupplung also extra manuell öffnen zu müssen, kann das Werkzeug damit unter Ausübung eines leichten Drucks beim Einsetzen in die erste, am weitesten aus dem Handstück herausragende, verriegelte Position gebracht werden.

Eine weitere Ausführungsform des erfindungsgemässen Handgriffes sieht vorteilhafterweise vor, dass die Verlängerungswelle durch eine zweite Feder in distaler Richtung beaufschlagt ist, wodurch eine Unterstützung der Versetzung des Aufnahmemechanismus in die für die Aufnahme des Werkzeugs vorgesehene Position und Stellung bzw. in die Stellung zur Entriegelung des Werkzeugs gewährleistet ist.

Der Aufnahmemechanismus ist erfindungsgemäss durch zumindest eine radial nach aussen hin elastisch beaufschlagte Lasche mit zumindest einer radial nach innen weisenden, komplementär zu einer Einkerbung des einzusetzenden Werkzeug-Schafts geformten Haltenoppe und durch zumindest eine die Lasche aussen umfassenden, axial fixierten Verriegelungshülse gebildet, deren innerer Durchmesser sich in proximaler Richtung auf den Handgriff hin verringert, wobei die Lasche in der äussersten distalen Position der Verlängerungswelle im Bereich des grösseren Durchmessers oder distal ausserhalb der Verriegelungshülse liegt und die Lasche in allen proximal anschliessenden Positionen der Verlängerungswelle im Bereich geringeren Durchmessers der Verriegelungshülse liegt. Dies gestattet die einfache und funktionssichere Kombination der Abstandsverstellung der Werkzeugspitze gegenüber dem Handgriff mit der manuellen Ent- oder Verriegelung des Werkzeugs in der Aufnahmeeinrichtung. Bei Betätigung der Abstandseinstellung über einen bestimmten Punkt hinaus wird automatisch die Entriegelung bzw. die Aufnahmebereitschaft für ein Werkzeug bewirkt.

Vorteilhafterweise und bei baulich einfacher und funktionssicherer Ausführung ändert sich der innere Durchmesser der Schliesshülse und/oder der Verriegelungshülse in Form einer konischen Abschrägung.

Gemäss einer alternativen Ausführungsform der Erfindung ist der Handgriff dadurch gekennzeichnet, dass der Aufnahmemechanismus durch zumindest eine radial nach aussen hin elastisch beaufschlagte Lasche mit zumindest einer radial nach innen weisenden, komplementär zu einer Einkerbung des einzusetzenden Werkzeugs geformten Haltenoppe und einer radial nach aussen hin weisenden Verriegelungsnoppe sowie durch zumindest eine die Lasche aussen umfassende, axial fixierte Verriegelungshülse gebildet ist, die zumindest eine innenliegende Ausnehmung für die Verriegelungsnoppe aufweist, welche Ausnehmung sich in der äussersten distalen Position der Verlängerungswelle genau oberhalb der Verriegelungsnoppe befindet und In allen proximal anschliessenden Positionen der Verlängerungswelle die Verriegelungsnoppe durch die Verriegelungshülse radial nach innen hin beaufschlagt und damit die Lasche radial nach innen hin gebogen ist. Auch diese Ausführungsform bietet den Vorteil der einfachen und funktionssicheren Kombination von Abstandsverstellung und manueller Ent- oder Verriegelung des Werkzeugs in der Aufnahmeeinrichtung wie oben bereits erläutert.

Bevorzugt ist zur Sicherstellung, dass der Aufnahmemechanismus in aufnahmebereiter Stellung verbleibt, solange kein Werkzeug komplett eingesetzt ist, ein Sperrelement in axialer Richtung nach distal zwischen die Laschen des Aufnahmemechanismus einschiebbar, vorzugsweise durch Beaufschlagung mittels eines Federelementes, und kann durch das distale Ende des Werkzeuges in proximaler Richtung aus dem Bereich der Laschen gedrückt werden.

Zur Lösung der weiteren Aufgabe umfasst das Werkzeugsystem einen Satz von Handgriffen mit eingebautem Telescoping-Mechanismus gemäss einem der vorhergehenden Absätze und vorzugsweise einen Satz von Handgriffen mit unterschiedlicher Länge der Führungshülse, wobei die Position des Aufnahmemechanismus für das Werkzeug relativ zum distalen Ende der Führungshülse für jeden Handgriff an dergleichen Längsposition liegt. Damit wird ein Set von verschieden langen Handgriffen bereitgestellt, entsprechend der jeweiligen grösseren oder kleineren distalen Lange der Führungshülse. FUr eine vorgegebene Anzahl von Werkzeugen identischer Schaftlänge, d.h. mit vereinfachter Lagerhaltung und verringerter Verwechslungsgefahr, kann mit der variablen Anzahl verschieden langer Handgriffe eine grosse Anzahl von unterschiedlichen Abständen von Werkzeugspitze und werkzeugführender Hand erzielt werden. Gleichzeitig bleibt die Möglichkeit des Teleskoping für jedes eingesetzte Werkzeug in jedem Handgriff erhalten, so dass neben dem standardmässig vorhandenem und gut eingeführtem Teleskoping ausserdem noch eine darüber hinausgehende Längenvariation für den Operateur gegeben ist, ohne die oben angegebenen Nachtelle in Kauf nehmen zu müssen.

Die Wechselvorgänge beschränken sich auf zwei oder 3 - sind also - trotz gestiegenen - nachteilfreien - Optionen nicht mehr als beim bekannten Teleskoping-System mit unterschiedlich langen Werkzeugschäften:
1) Handgriff weg,
2) Neuer Handgriff ran
3) Betätigung des Teleskoping-Mechanismus auf die gewünschte Länge des neuen Werkzeugs.

Der scheinbare Nachteil, des zur Verfügungstellens eines Sets von relativ voluminösen Handgriffen wird aus erfindungsgemässer Sicht dadurch mehr als kompensiert, dass die Herstellung der Werkzeuge an sich oftmals - weil mikromechanisch - sehr aufwendig und teuer ist (oftmals sogar vergleichbar mit der Herstellung eines Handgriffs), dass aber andererseits die Verwendungssicherheit und Manipulationssicherheit unter Operationsbedingungen wesentlich vereinfacht ist. So behält der Chirurg beispielsweise nach dem Lösen der Verbindung zwischen dem Werkzeug und dem Handgriff das (einzige kleine) Werkzeug, das er aus dem Handgriff entfernt, in seiner Hand und kann problemlos und griffsicher den Handgriff seiner Assistentin übergeben, die ebenso griffsicher den neuen Handgriff reichen kann. Unabsichtliches Fallenlassen von Teilen des Systems (Werkzeugen) ist somit sicher verhindert, was die Operationssicherheit deutlich erhöht.

Bevorzugt ist dabei die Position des Aufnahmemechanismus für das Werkzeug bzw. dessen proximalen Werkzeug-Ende relativ zum distalen Ende der Führungshülse durch den Teleskoping-Mechanismus mittels eines manuell betätigbaren Einstellmechanismus axial - wie an sich bekannt - in diskreten Schritten verstellbar. Unter Erhaltung der oben genannten Merkmale kann dabei für jede Handgrifflänge nochmals eine begrenzte Variation des Abstandes der distalen Werkzeugspitze gegenüber der werkzeugführenden Hand erzielt werden, wobei aber durch die begrenzte Abstandsverstellung - wie an sich bekannt - nur kleine, vernachlässigbare bzw. beherrschbare Probleme mit der seitlichen Führung des Werkzeugs zu befürchten sind.

Gemäss einer bevorzugten Ausführungsform ist vorgesehen, dass der Einstellmechanismus für das Teleskoping an jedem Handgriff an der gleichen Relativ-Position zum Gehäuse vorgesehen ist und sich distal daran eine Verlängerungswelle mit dem Aufnahmemechanismus für das Werkzeug anschliesst. Dies gestattet die gleichartige Ausführung jedes Handgriffes mit Ausnahme der Länge der Führungshülse, was die Herstellung und Wartung des Werkzeugsystems sowie die Bedienung des Handgriffes zur Abstandsveränderung bzw. zum Einsetzen oder Entnehmen des Werkzeugs wesentlich vereinfacht.

Der Handgriff und das Werkzeugsystem wie in den vorhergehenden Absätzen beschrieben sind besonders vorteilhaft einsetzbar mit einem medizinischen Werkzeug, dessen Schaft über den Verstellbereich des Teleskoping-Mechanismus axial beabstandete Markierungen, vorzugsweise radiale Ringe, aufweist, so dass die axiale Verstellung exakt durchgeführt und die axiale Position des Werkzeuges genau überprüft werden können. Damit wird die Handhabung beim Einsetzen und Verstellen des Werkzeuges innerhalb des Handgriffes erleichtert, insbesondere, wenn vorzugsweise am Schaft eine weitere Markierung proximal der übrigen Markierungen vorgesehen ist, vorzugsweise ein breiter umlaufender Ring, dessen Breite derart gewählt ist, dass die weitere Markierung so lange sichtbar ist, wie das Werkzeug in einer nicht verriegelten Position ist. Wenn das Werkzeug in verriegelter Stellung ist, wird diese weitere Markierung vom Handstück, vorzugsweise von dessen distaler Führungshülse, abgedeckt.

Weitere Vorteile, Merkmale und Einzelheiten der Erfindung ergeben sich aus der nachfolgenden Beschreibung, in der unter Bezugnahme auf die Zeichnungen Ausführungsbeispiele der Erfindung beschrieben sind. Dabei können die in den Ansprüchen und in der Beschreibung erwähnten Merkmale jeweils einzeln für sich oder in beliebiger Kombination erfindungswesentlich sein.

Die Bezugszeichenliste ist wie auch der technische Inhalt der Patentansprüche und Figuren Bestandteil der Offenbarung. Die Figuren werden zusammenhängend und übergreifend beschrieben. Gleiche Bezugszeichen bedeuten gleiche Bauteile, Bezugszeichen mit unterschiedlichen Indices geben funktionsgleiche oder ähnliche Bauteile an.

Es zeigen dabei:
- Fig. 1: eine erste Ausführungsform eines erfindungsgemässen Handgriffs für ein medizinisches Werkzeugsystem, als Teil eines Sets mit einer Mehrzahl von Handgriffen, mit kurzer Führungshülse für das eingesetzte Werkzeug,
- Fig. 2: ist eine Abbildung eines weiteren erfindungsgemässen Handgriffs aus dem erfindungsgemässen Set von Handgriffen, hier mit einer längeren Führungshülse für das Werkzeug als in Fig. 1,
- Fig. 3: in vergrössertem Massstab den Handgriff der Fig. 1 mit verriegeltem Werkzeug und in einer Stellung mit am weitesten eingefahrenem Werkzeug-Schaft (kurze Position),
- Fig. 4: zeigt den Handgriff der Fig. 1 mit kurzer Führungshülse, mit offenem Aufnahmemechanismus und erst teilweise in die Führungshülse eingeführtem Werkzeug-Schaft,
- Fig. 4a: zeigt eine Draufsicht auf das distale Ende einer speziellen Ausführungsform eines erfindungsgemässen Werkzeuges,
- Fig. 5: zeigt den Handgriff der Fig. 1 im Zustand mit komplett eingesetztem Werkzeug-Schaft, der jedoch noch nicht verriegelt ist,
- Fig. 6: stellt den Zustand des Handgriffs der Fig. 1 mit verriegeltem Werkzeug und in seiner am weitesten ausgefahrenen Teleskoping-Stellung (lange Position),
- Fig. 7a: ist eine Darstellung in vergrössertem Massstab einer weiteren Ausführungsform eines erfindungsgemässen Handgriffes mit Schliesshülse mit unterschiedlichen Konus in der am weitesten proximalen Position der Verlängerungswelle, und
- Fig. 7b: ist eine Darstellung entsprechend der Fig. 7a in der am weitesten distalen Position der Verlängerungswelle.

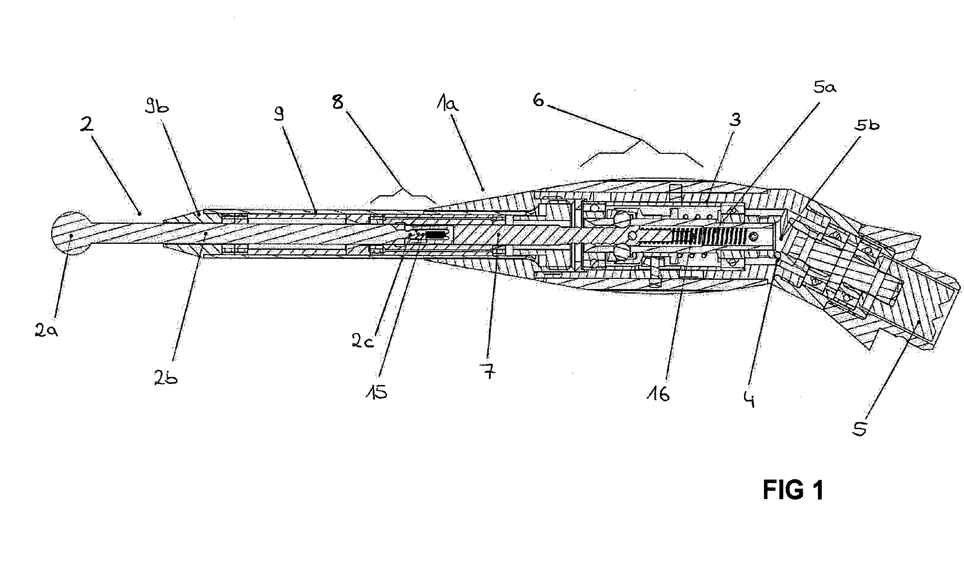

Fig. 1 zeigt einen Handgriff 1 für ein medizinisches Werkzeugsystem, in welchen Handgriff 1 ein chirurgisches oder zahnärztliches Werkzeug 2 eingesetzt werden kann. Der Handgriff 1 beinhaltet in seinem Gehäuse 3 einen Rotationsantrieb 4, beispielsweise das Ende 5a einer Welle 5, die von einem externen oder im Handgriff eingebauten Druckluftantrieb, Elektromotor oder anderem Motor ausgeht. Bei gebogenen oder abgeknickten Handgriffen wie in Fig. 1 beinhaltet der Rotationsantrieb 4 auch eine Umlenkung 5b, vorzugsweise über schräg verzahnte Stirnräder, ein Kardangelenk oder dergleichen.

Am distalen Ende des Rotationsantriebes 4, der vorzugsweise an einer axial fixierten Position innerhalb des Gehäuses 3 des Handgriffes 1 und damit auch immer in der gleichen Position relativ zur werkzeugführenden Hand des Chirurgen oder Zahnarztes liegt, ist ein manuell betätigbarer Einstellmechanismus 6 für eine Verlängerungswelle 7 der Welle 5 vorgesehen, mit welchem Einstellmechanismus 6 der axiale Abstand des distalen Endes der Verlängerungswelle 7 relativ zum fixen Ende 5a der Welle 5 des Rotationsantriebes 4 manuell eingestellt werden kann (Teleskoping). Vorzugsweise erfolgt die Abstandseinstellung mittels des Einstellmechanismus 6 in diskreten Schritten.

Am distalen Ende der Verlängerungswelle 7 ist ein vorzugsweise ebenfalls manuell ver- und entriegelbaren Aufnahmemechanismus 8 für das medizinische Werkzeug 2 vorgesehen, dessen vom distalen Ende 2a ausgehender Schaft 2b in einer vom Handgriff 1 distal ausgehenden, axial fixierten Führungshülse 9 für das Werkzeug 2 aufgenommen und dadurch seitlich abgedeckt und auch geführt ist. Das Werkzeug 2 ist damit durch Wirkung des Rotationsantriebes 4, des daran angeschlossenen Einstellmechanismus 6, der sich mit dem Rotationsantrieb 4 mitdreht, und mittels der ebenfalls mitdrehenden Verlängerungswelle 7, deren distales Ende durch den Aufnahmemechanismus 8 gebildet ist, in Drehung versetzbar.

Durch die Abstandsverstellung der Verlängerungswelle 7 relativ zur Welle 5 mittels des Einstellmechanismus 6 wird gleichzeitig auch der Abstand des distalen Endes 2a des Werkzeugs 2 gegenüber dem Handgriff 1 und damit der werkzeugführenden Hand auf die gewünschte Länge eingestellt. Da aber der Einstellmechanismus 6 nur eine beschränkte Längenänderung zulässt (minimal: kurze Position maximal: lange Position), muss für gewünschte grössere Einstellbereiche eine zusätzliche Lösung vorgesehen sein. Diese Lösung besteht darin, dass das Werkzeugsystem eine Mehrzahl von Handgriffen 1 mit unterschiedlicher Länge, vorzugsweise unterschiedlicher Länge der Führungshülse 9 und der darin geführten Verlängerungswelle 7 aufweist.

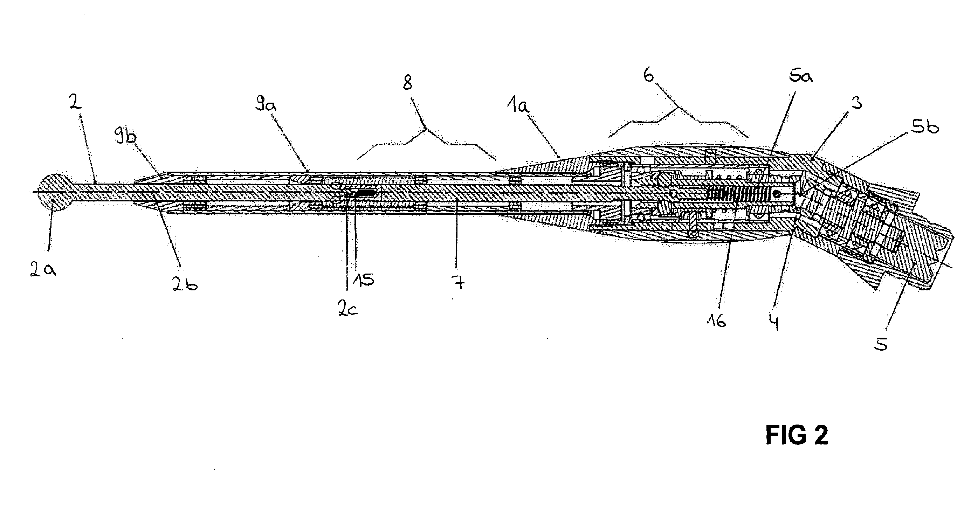

Fig. 2 stellt einen derartigen weiteren Handgriff 1a dar, der eine längere Führungshülse 9a als jene des kürzeren Handgriffs 1 aufweist. Die Verlängerungswelle 7a ist im gleichen Mass verlängert wie die Führungshülse 9a, so dass sich für alle unterschiedlich langen Handgriffe 1, 1a der Aufnahmemechanismus 8 für das Werkzeug 2 relativ zum distalen Ende der Führungshülse 9, 9a im gleichen Abstand befindet, wenn der Einstellmechanismus 6 in der gleichen Stellung ist (wie in den Fig. 1 und 2). Mit einem derartigen Werkzeugsystem kann mit einem Satz von Werkzeugen 2 mit gleicher Länge eine grosse Anzahl an unterschiedlichen Abständen zwischen dem jeweils unterschiedlich ausgeführten distalen Ende 2a des Werkzeugs 2 und der werkzeugführenden Hand realisiert werden, je nach Bedarf für die konkrete durchzuführende Behandlung.

Vorzugsweise liegen sowohl der Rotationsantrieb 4 sowie alle daran anschliessenden Elemente wie Einstellmechanismus 6 und Verlängerungswelle 7, 7a bei jedem einzelnen Handgriff 1, 1a der Mehrzahl der Handgriffe des Werkzeugsystems an der gleichen Position innerhalb des Gehäuses 3, was aufgrund der gleichartigen Konstruktion aller Handgriffe 1, 1a die Fertigung, Bedienung und Wartung des Systems wesentlich vereinfacht.

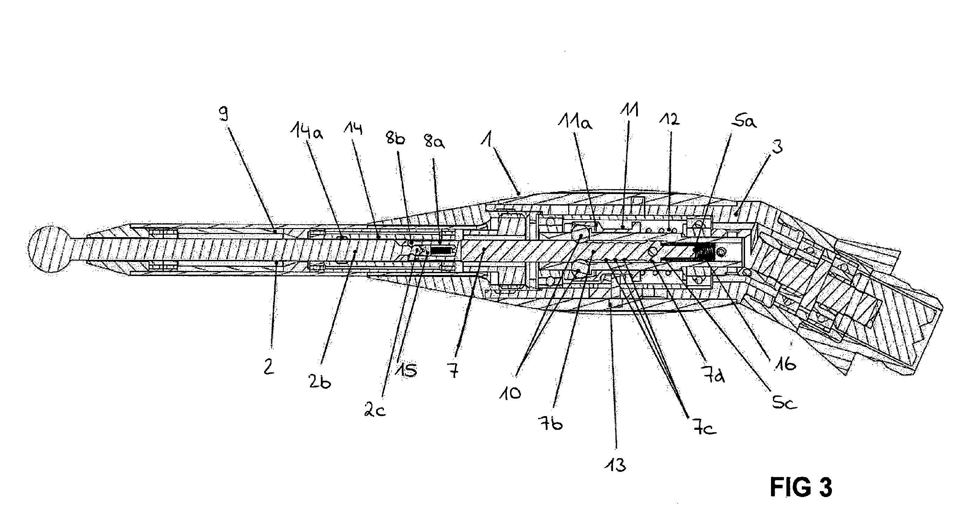

In Fig. 3 ist in vergrössertem Massstab der Bereich des Handgriffs 1 der Fig. 1 mit kurzer Führungshülse 9 und kurzer Verlängerungswelle 7 dargestellt, der den Einstellmechanismus 6 als auch den Aufnahmemechanismus 8 für das Werkzeug 2 umfasst.

Der Einstellmechanismus 6 umfasst den proximale Endbereich 7b der Verlängerungswelle 7, welches in einer zentralen Sackbohrung 5c des distalen Endes 5a der Welle 5 verschiebbar aufgenommen ist. Vorzugsweise ist zwischen dem Endbereich 7b und dem Boden der Sackbohrung 5c eine Feder 16 eingesetzt, welche die Verlängerungswelle 7 in distaler Richtung elastisch mit einer Kraft beaufschlagt.

Entlang des proximalen Endbereiches 7b ist eine der gewünschten Anzahl von Einstellmöglichkeiten entsprechende Mehrzahl von axial beabstandeten Einkerbungen 7c vorgesehen. Ein oder vorzugsweise eine Mehrzahl von Haltekugeln 10 können zur axialen Fixierung der Verlängerungswelle 7 relativ zur Welle 5 in diese Einkerbungen 7c eingreifen und hineingepresst werden, wofür eine Schliesshülse 11 vorgesehen ist. Diese Schliesshülse 11 weist einen distalen Endbereich 11 a mit sich vorzugsweise konisch nach distal vergrösserndem Innendurchmesser auf. Die axiale Fixierung der Haltekugeln 10 innerhalb des Gehäuses 3 kann durch eine separate Hülse bewerkstelligt werden oder - wie im dargestellten Ausführungsbeispiel - durch Lagerung der Haltekugeln 10 in Öffnungen des distalen Endes 5a der Welle 5.

Die Schliesshülse 11 wird durch eine Feder 12 elastisch mit einer Kraft beaufschlagt, durch welche der Abschnitt mit geringerem Innendurchmesser des Endabschnittes 11a mit den Haltekugeln 10 zusammenwirkt und diese in die Einkerbungen 7c presst. Um die Haltekugeln 10 freizugeben und damit eine axiale Verschiebung der Verlängerungswelle 7 zu ermöglichen, kann die Schliesshülse 11 mittels eines von aussen betätigbaren Betätigungselementes 13 am Handgriff 1 gegen die Wirkung der Feder 12 derart verschoben werden, dass der Abschnitt des Endbereiches 11a mit grösserem Durchmesser mit der Position der Haltekugeln 10 in Überstimmung kommt.

Die Verschiebung der Verlängerungswelle 7 kann dabei entweder durch die Wirkung der Feder (16) zwischen deren Endbereich 7b und dem Boden der Bohrung 5c in der Welle 5 oder durch das Einschieben des Werkzeugs 2 in die Führungshülse 9 und damit Einwirken auf den Aufnahmemechanismus 8 bewirkt werden.

Wie des Weiteren noch aus Fig. 3 hervorgeht, ist die vorzugsweise Ausführung des Aufnahmemechanismus 8 mit zumindest einer radial nach aussen hin elastisch beaufschlagten Lasche 8a mit einem sowohl radial nach aussen als auch radial nach innen hin verdickten Ende 8b, das somit eine radial nach innen weisende Haltenoppe als auch eine nach aussen weisende Verriegelungsnoppe bildet. Der nach innen weisende Teil des Endes 8b ist vorzugsweise komplementär zu einer Einkerbung im proximalen Ende 2c des einzusetzenden Werkzeugs 2 geformt.

Da die Drehmomentübertragung, d.h. die radiale Kraftübertragung zwischen Rotationsantrieb 4 und Werkzeug 2, so ausgelegt ist, dass das Drehmoment über den maximalen Durchmesser (möglichst weit von der Rotationsachse entfernt) übertragen wird, sind vorzugsweise die fixierenden Teile des Aufnahmemechanismus 8, insbesondere die Laschen 8a, möglichst zentrisch positioniert. Dadurch wird die radiale Kraftübertragung verbessert bzw. nicht gestört.

Eine axial relativ zum Handgriff 1 und zur Führungshülse 9 fixierte Verriegelungshülse 14 umfasst aussen in jeder beliebigen axialen Position der Verlängerungswelle 7 und damit auch des Aufnahmemechanismus 8 jede der Laschen 8a. Der innere Durchmesser der Verriegelungshülse 14 ist dabei derart gewählt, dass er für alle axialen, durch den Einstellmechanismus 6 einstellbaren Positionen der Verlängerungshülse 7 die Laschen 8a radial nach innen presst, mit Ausnahme der ganz äussersten Position der Verlängerungswelle 7, für deren Einstellung aber nachfolgend noch erläuterte spezielle Vorkehrungen getroffen werden müssen. In denjenigen Positionen, die durch die Einkerbungen 7c definiert sind, bleiben die Laschen 8a nach innen gehalten, greifen damit mit ihrem Ende 8b in die Einkerbung 2c am Werkzeug ein und halten dieses damit verriegelt im Handgriff 1.

Neben den Einkerbungen 7c weist der Endbereich 7b der Verlängerungswelle 7 noch zumindest eine Einkerbung 7d an einer am weitesten proximalen Position auf. Diese am weitesten proximale Einkerbung 7d ist gegenüber den anderen, sich weiter distal anschliessenden Einkerbungen 7c radial weiter aussenliegend vorgesehen, so dass die Schliesshülse 11 noch ein Stück weiter gegen die Feder 12 verschoben werden muss als für die normale Längenverstellung des Werkzeugs 2. Damit ist eine unabsichtliche Entriegelung des Werkzeugs 2 mit grosser Sicherheit ausgeschlossen.

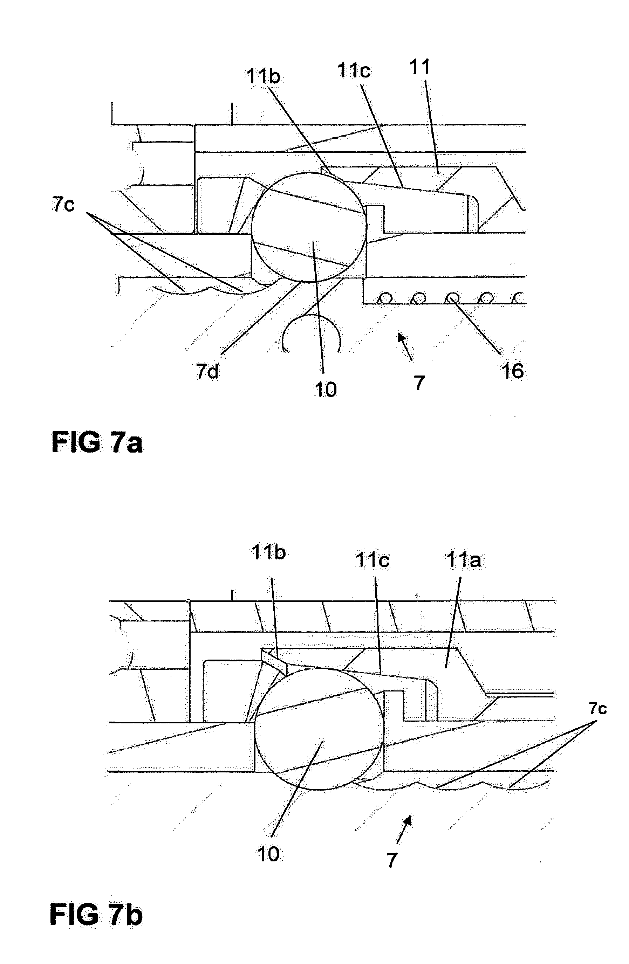

Die mit der oder jeder Haltekugel 10 zusammenwirkende Innenwandung des distalen Abschnittes 11a der Schliesshülse 11 ist, wie auch in den Fig. 7a und 7b dargestellt ist, zumindest im Bereich 11 b des grösseren inneren Durchmessers konisch geformt. Der Konuswinkel im Bereich 11 b des grösseren inneren Durchmessers ist dabei grösser als im Bereich 11 c des kleineren inneren Durchmessers. Dadurch kommt die Haltekugel 10, wenn sie in der am weitesten proximalen und radial am weitesten aussen liegenden Einkerbung 7d der Verlängerungswelle 7 liegt, in Kontakt mit einem Konusbereich der Schliesshülse 11 mit sich stärker bzw. mit einem steileren Winkel öffnenden Innendurchmesser. Beim Einschieben des Werkzeuges 2 in den Aufnahmemechanismus 8 kann mit einer noch von Hand zu überwindenden Kraft die Schliesshülse 11 gegen die Wirkung der Feder 12 verschoben werden. Ohne die Kupplung also extra manuell öffnen zu müssen, kann das Werkzeug 2 damit unter Ausübung eines leichten Drucks in die erste, am weitesten aus dem Handstück 1, 1a herausragende, verriegelte Position gebracht werden.

Sobald sich das Werkstück aber in dieser Position befindet, kommt die oder jede Haltekugel 10 in den Bereich der radial weiter innen liegenden Einkerbungen 7c der Verlängerungswelle 7 und auch in den Bereich 11c der Schliesshülse 11 mit geringerem Innendurchmesser. Der Innendurchmesser des distalen Abschnittes 11a der Schliesshülse 11 weist in diesem Bereich 11 c eine konische Form mit geringerem Öffnungswinkel auf, so dass eine axiale Krafteinwirkung durch das Werkzeug 2 allein keine Verschiebung der Schliesshülse 11 mehr bewirken kann. Zur Verschiebung der Schliesshülse 11 und zur Verstellung des Teleskoping-Mechanismus ist nunmehr die manuelle Betätigung notwendig.

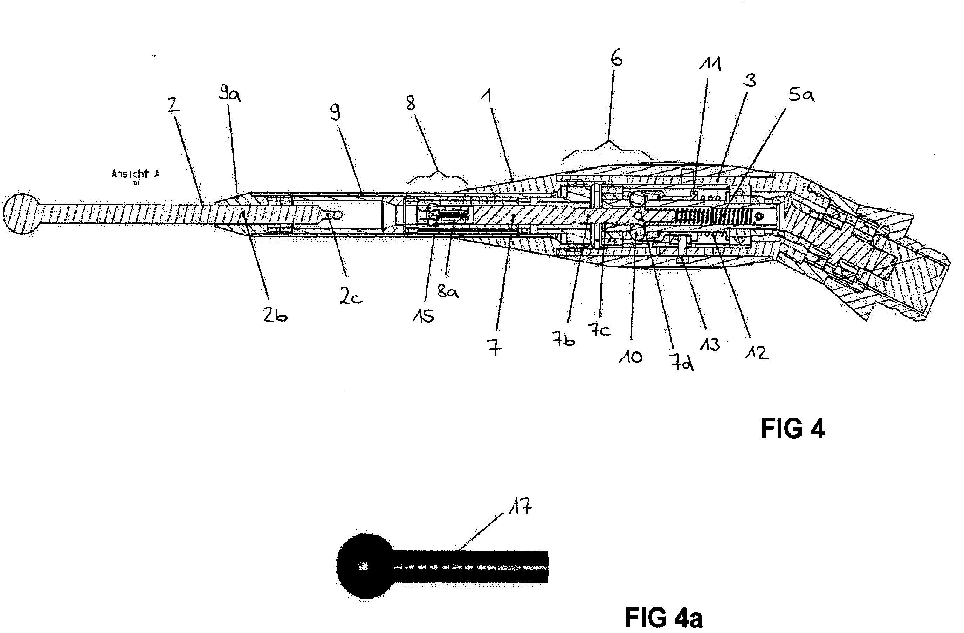

In dieser Stellung, die in Fig. 4 dargestellt ist, ist die Verlängerungswelle 7 in ihrer am weitesten ausgefahrenen Position und die Enden 8b jeder Lasche 8a gelangen in einen Bereich 14a der Verriegelungshülse 14 mit grösserem Durchmesser. Dieser Bereich 14a grösseren Durchmessers kann durch eine innen umlaufende Nut oder innen an den Umfangspositionen der Laschen 8a vorgesehenen Dellen gebildet sein, oder auch dadurch, dass sich der innere Durchmesser der Verriegelungshülse 14 in Form einer konischen Abschrägung ändert, und sich vorzugsweise der innere Durchmesser sich in proximaler Richtung auf den Handgriff 1 hin verringert.

Fig. 4 jedenfalls zeigt den Handgriff 1 der Fig. 1 in einem Zustand, in welchem das proximale, in der Führungshülse 9 befindliche Ende 2c des Werkzeugs 2 noch nicht - oder nicht mehr - mit dem Aufnahmemechanismus 8 verbunden und darin lösbar geklemmt ist.

Des Weiteren ist in Fig. 4a eine vorteilhafte Ausführungsform dieses Werkzeuges 2 dargestellt. Zur axialen Orientierung sind die Werkzeuge 2 über den Verstellbereich des Teleskoping-Mechanismus mit axial beabstandeten Markierungen, vorzugsweise radialen Ringen 17 versehen.

Bevorzugt ist auch eine weitere Markierung proximal der Markierungen 17 vorgesehen, beispielsweise ein breiter umlaufender Ring, der so lange sichtbar ist, wie das Werkzeug 2 nicht verriegelt ist und der im distalen Führungshülsenende 9b verschwindet, sobald das Werkzeug 2 korrekt gespannt ist. Dazu ist typischerweise die Breite der weiteren Markierung derart gewählt, dass sie eben so lange sichtbar ist, wie das Werkzeug 2 in einer nicht verriegelten Position ist, und dass die weitere Markierung vom Handstück 1, 1a, vorzugsweise der distalen Führungshülse 9 bzw. deren Ende 9b, abgedeckt ist, wenn das Werkzeug 2 in verriegelter Stellung ist. Vorzugsweise sind die Markierungen am Werkzeug 2 durch Laserbeschriftung hergestellt.

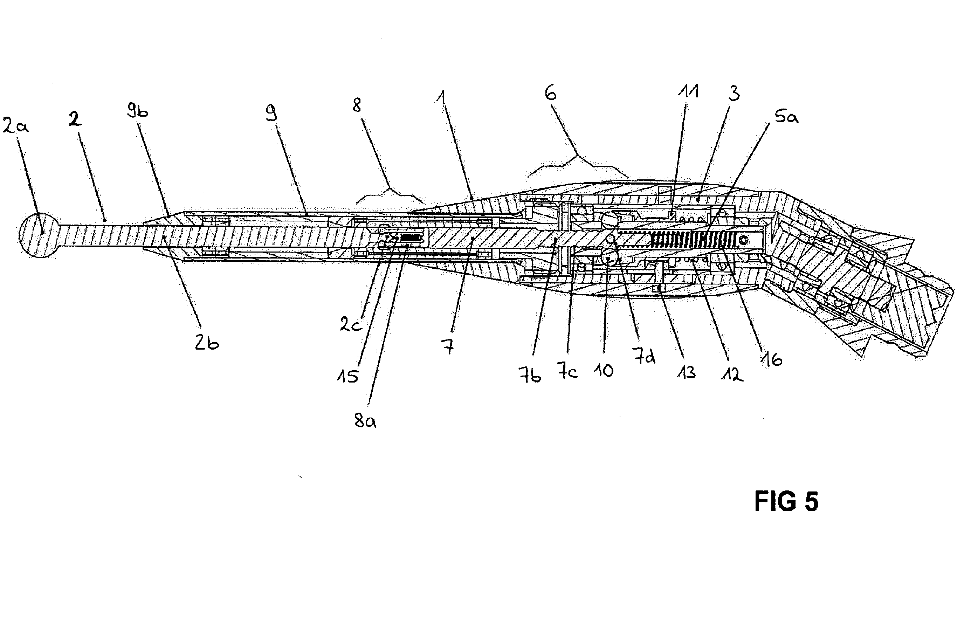

Die Fig. 5 ist eine Darstellung, die sich bei weiterem Einschieben des Werkzeugs 2 in die Führungshülse 9 ergibt. Hier ist die Verlängerungswelle 7 zwar immer noch in der äussersten Position mit den Haltekugeln 10 in den radial äussersten und proximal am weitesten im Inneren des Handgriffes 1 liegenden Einkerbungen 7c, das proximale Ende 2c des Werkzeugs 2 ist jedoch bereits in der zu verriegelnden Stellung in Relation zum Aufnahmemechanismus 8.

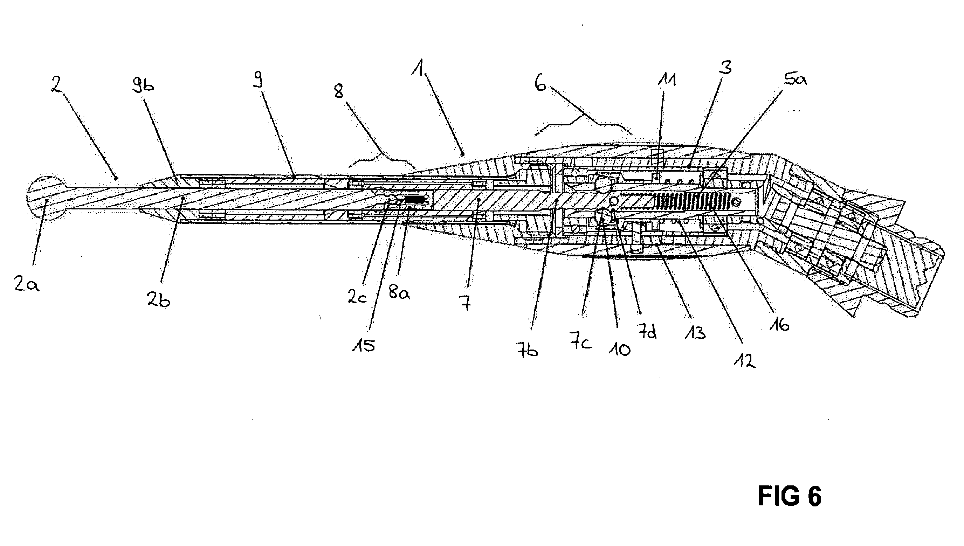

Beim weiteren Einschieben des Werkzeugs 2 in die Führungshülse 9 wird, wie in Fig. 6 dargestellt ist, die Verlängerungswelle 7 gegen die Wirkung der Feder in der Sackbohrung 5c der Welle 5 in den Handgriff 1 hinein bewegt, wodurch die oder jede erste proximale Einkerbung 7b mit der Position der Haltekugeln 10 in Übereinstimmung kommen und die Schliesshülse 11 durch die Wirkung der Feder 12 ein Stück in Richtung auf das distale Ende des Handgriffes 1 hin verschoben werden kann. Dadurch wiederum werden die Haltekugeln 10 in die erste proximale Einkerbung 7b gedrückt und die Verlängerungswelle 7 in dieser Stellung fixiert. Gleichzeitig mit den Hineinschieben der Verlängerungswelle 7 in die Sackbohrung 5c der Welle 5b wird auch der Aufnahmemechanismus 8 derart betätigt, dass die verdickten Enden 8b der Laschen 8a in den näher dem Handgriff 1 liegenden Bereich der Verriegelungshülse 9 gelangen, wo sie aufgrund des dort geringeren Innendurchmessers der Verriegelungshülse 9 radial nach innen hin gedrückt werden und in den komplementär geformten Endabschnitt des Werkzeugs 2 eingreifen. Das Werkzeug 2 ist damit im Handgriff 1, in einer axial gegenüber der Verlängerungswelle 7 fixierten, gegenüber dem Handgriff 1 und der Führungshülse 9 jedoch über den Einstellmechanismus 6 in vorzugsweise diskreten Schritten axial verstellbaren Positionen verriegelt.

Im Bereich des Aufnahmemechanismus 8 kann bei einer bevorzugten Ausführungsform eines erfindungsgemässen Handgriffs 1, 1a zusätzlich eine Sperre eingebaut sein, die verhindert, dass sich die Verlängerungswelle 7 beim Einschieben eines Werkzeuges 2 axial bewegen kann, ohne das Werkzeug 2 komplett zu spannen, was eine Fehlfunktion der Kupplung bewirken könnte. Dazu kann ein Sperrelement 15 in axialer Richtung nach distal zwischen die Laschen 8a des Aufnahmemechanismus 8 durch die Wirkung einer Feder eingeschoben werden. Durch das distale Ende 2c des Werkzeuges 2 wird das Sperrelement 15 beim Einschieben des Werkzeuges 2 gegen die Wirkung der Feder wieder aus dem Bereich der Laschen 8a gedrückt, die damit ihre Verriegelungsstellung einnehmen können. Beim Entnehmen des Werkzeuges 2 hingegen wird das Sperrelement 15 zwischen die Laschen 8a hinein bewegt. Diese sind nun in der Nut der Verriegelungshülse 14a fixiert und können sich erst wieder axial bewegen, wenn das Werkzeugende 2c das Sperrelement 15 nach hinten schiebt.

**Bezugszeichenliste**

| | | | |
|---|---|---|---|
| 1, 1a | Handgriff | 11a | Endbereich der Schliesshülse |
| 2 | Werkzeug | 12 | Feder der Schliesshülse |
| 2a | Distales Werkzeugende | 13 | Betätigungselement |
| 2b | Werkzeugschaft | 14 | Verriegelungshülse |
| 2c | Proximales Werkzeugende | 14a | Nut bzw. Ausnehmung in der Verriegelungshülse |
| 3 | Gehäuse | 15 | Sperrelement |
| 4 | Rotationsantrieb | 16 | Feder der Verlängerungswelle |
| 5 | Welle | 17 | Markierungsringe |
| 5a | Wellenende | | |
| 5b | Umlenkung | | |
| 5c | Sackbohrung | | |
| 6 | Einstellmechanismus | | |
| 7,7a | Verlängerungswelle | | |
| 7b | Endbereich der Verlängerungswelle | | |
| 7c | Distale Einkerbungen | | |
| 7d | Proximale Einkerbungen | | |
| 8 | Aufnahmemechanismus | | |
| 8a | Laschen | | |
| 8b | Laschenenden | | |
| 9, 9a | Führungshülse | | |
| 9b | Distales Führungshülsenende | | |
| 10 | Haltekugeln | | |
| 11 | Schliesshülse | | |

## Patentansprüche

1. Handgriff (1, 1 a) für ein medizinisches Werkzeugsystem, mit einem Gehäuse (3), einem Rotationsantrieb (4) an einer axial fixierten Position innerhalb des Gehäuses (3), einem vorzugsweise manuell verund entriegelbaren Aufnahmemechanismus (8) für ein in den Handgriff (1, 1a) einsetzbares und durch den Rotationsantrieb (4) antreibbares medizinisches Werkzeug (2), sowie mit einer vom Handgriff (1, 1a) distal ausgehenden, axial fixierten Führungshülse (9, 9a) für das Werkzeug (2) und mit einem manuell betätigbaren, einstellbaren Teleskoping-Mechanismus, dessen manuell betätigbare Einstellmechanismus (6) durch eine Mehrzahl von axial beabstandeten Einkerbungen (7c, 7d) im proximalen Ende (7b) der Verlängerungswelle (7), durch zumindest eine im Gehäuse (3) axial fixiert aber radial beweglich gehaltene Haltekugel (10) und eine axial durch eine Feder (12) beaufschlagte, die Haltekugeln (10) umfassende und manuell betätigbare Schliesshülse (11, 11a) gebildet ist, deren innerer Durchmesser sich in axialer Richtung ändert, wobei ein kleinerer Durchmesser die oder jede Haltekugel (10) in jeweils eine Einkerbung (7c, 7d) der Verlängerungswelle (7) drückt und ein grösserer Durchmesser die oder jede Haltekugel (10) aus der jeweiligen Einkerbung (7c, 7d) freigibt, **dadurch gekennzeichnet, dass** die am weitesten proximale Einkerbung (7d) an der Verlängerungswelle (7) für die kurze Position radial weiter aussen liegt als alle sich distal anschliessenden Einkerbungen (7c) für längere bzw. für die lange Position.

2. Handgriff nach Anspruch 1, **dadurch gekennzeichnet, dass** die mit der oder jeder Haltekugel (10) zusammenwirkende Innenwandung der Schliesshülse (11, 11a) zumindest im Bereich (11 b) des grösseren inneren Durchmessers konisch geformt ist, wobei der Konuswinkel im Bereich (11 b) des grösseren inneren Durchmessers grösser ist als im Bereich (11 c) des kleineren inneren Durchmessers.

3. Handgriff nach einem der Ansprüche 1 oder 2, **dadurch gekennzeichnet, dass** die Verlängerungswelle (7) durch eine zweite Feder (16) in distaler Richtung beaufschlagt ist.

4. Handgriff nach einem der Ansprüche 1 bis 3, **dadurch gekennzeichnet, dass** der Aufnahmemechanismus (8) durch zumindest eine radial nach aussen hin elastisch beaufschlagten Lasche (8a) mit zumindest einer radial nach innen weisenden, komplementär zu einer Einkerbung des einzusetzenden Werkzeugs geformten Haltenoppe (8b) und durch zumindest eine die Lasche (8a) aussen umfassenden, axial fixierten Verriegelungshülse (14) gebildet ist, deren innerer Durchmesser sich in proximaler Richtung auf den Handgriff (1, 1a) hin verringert, wobei die Lasche (8a) in der äussersten distalen Position der Verlängerungswelle (7) im Bereich des grösseren Durchmessers (14a) oder distal ausserhalb der Verriegelungshülse (14) liegt und die Lasche (8a) in allen proximal anschliessenden Positionen der Verlängerungswelle (7) im Bereich geringeren Durchmessers der Verriegelungshülse (14) liegt.

5. Handgriff nach Anspruch 4, **dadurch gekennzeichnet, dass** ein Sperrelement (15) in axialer Richtung nach distal zwischen die Laschen (8a) des Aufnahmemechanismus (8) einschiebbar ist, vorzugsweise durch Beaufschlagung mittels eines Federelementes, und durch das distale Ende (2c) des Werkzeuges (2) in proximaler Richtung aus dem Bereich der Laschen (8a) gedrückt werden kann.

6. Handgriff nach einem der Ansprüche 1 bis 5, **dadurch gekennzeichnet, dass** sich der innere Durchmesser der Schliesshülse (11, 11 a) und/oder der Verriegelungshülse (14) in Form einer konischen Abschrägung ändert.

7. Medizinisches Werkzeugsystem mit einem Handgriff (1, 1 a), der mit Je einem Werkzeug (2) aus einem Satz von Werkzeugen unterschiedlicher Ausführung des distalen Endes (2a) aber identischer Dimensionierung und Ausführung des daran anschliessenden Schaftes (2b) und des proximalen Endes (2c) koppelbar ist, wobei das proximale Ende (2c) des Werkzeugs in einem Aufnahmemechanismus (8) im Handgriff (1, 1a) lösbar geklemmt und mittels eines den Aufnahmemechanismus (8) beinhaltenden Rotationsantriebes (4) In Drehung versetzbar ist, und wobei der Handgriff (1, 1a) eine distale Führungshülse (9, 9a) für den Schaft (2b) des Werkzeugs (2) aufweist und wobei der Aufnahmemechanismus mit einem Teleskoping-Mechanismus versehen ist, der eine axiale Variation der Relativposition von Werkzeug (2) relativ zur Führungshülse (9,9a) von einer kurzen Position zur einer langen Position gestattet, **dadurch gekennzeichnet, dass** der Handgriff nach einem der Ansprüche 1 bis 6 ausgeführt ist.

8. Werkzeugsystem nach Anspruch 7, **dadurch gekennzeichnet, dass** das System einen Satz Handgriffen (1, 1a) mit unterschiedlicher Länge der Führungshülse (9, 9a) umfasst, wobei die Position des Aufnahmemechanismus (8) für das Werkzeug (2) in seiner kurzen oder In seiner langen Position relativ zum distalen Ende (9b) der Führungshülse (9, 9a) für jeden Handgriff (1, 1a) an der gleichen kurzen oder langen Position liegt.

9. Werkzeugsystem nach Anspruch 8, **dadurch gekennzeichnet, dass** beim Teleskoping-Mechanismus die Position des Aufnahmemechanismus (8) für das Werkzeug (2) relativ zum distalen Ende (9b) der Führungshülse (9, 9a) mittels eines manuell betätigbaren Einstellmechanismus (6) axial in mehreren diskreten Schritten zwischen der kurzen und der langen Position verstellbar ist.

10. Werkzeugsystem nach Anspruch 9, **dadurch gekennzeichnet, dass** der Einstellmechanismus (6) für Jeden Handgriff (1, 1a) relativ zum Aussengehäuse (3) des Handgriffs (1,1a) an der gleichen Position vorgesehen ist und sich distal daran eine vorzugsweise jeweils unterschiedlich lange Verlängerungswelle (7) mit dem Aufnahmemechanismus (8) für das Werkzeug (2) anschliesst.

## Claims

1. A handle (1, 1a) for a medical tool system, with a housing (3), a rotational drive (4) on an axially affixed position within the housing (3), a preferably manually lockable and unlockable uptake mechanism (8) for a medical tool (2) that can be inserted into the handle (1, 1a) and that can be driven by the rotational drive (4), and with an axially fixed guide sleeve (9, 9a), which extends distally from the handle (1, 1a), for the tool (2), and with an adjustable telescoping mechanism, which is manually activateable, whose manually activateable adjustment mechanism (6) is formed by a plurality of axially spaced-apart indentations (7c, 7d) in the proximal end (7b) of the extension shaft (7), by means of at least one retaining ball (10) which is held in the housing (3) so as to be axially fixed but radially movable, and by means of a closing sleeve (11, 11a), which is axially impinged on by a spring (12), encompasses the retaining balls (10) and is manually activateable, with the internal diameter of said closing sleeve (11, 11a) changing in the axial direction, wherein a smaller diameter presses the or each retaining ball (10) in each case into a respective indentation (7c, 7d) of the extension shaft (7) and a larger diameter releases the or each retaining ball (10) from the respective indentation (7c, 7d), **characterised in that** the furthest proximal indentation (7d) on the extension shaft (7) for the short position is situated radially further outwards than all the distally following indentations (7c) for longer positions or for the longer position.

2. The handle according to claim 1, **characterised in that** the internal wall of the closing sleeve (11, 11a), which interacts with the or each retaining ball (10), at least in the region (11b) of the larger internal diameter, is conically formed, wherein the cone angle in the region (11b) of the larger interior diameter is greater than in the region (11c) of the smaller internal diameter.

3. The handle according to one of claims 1 or 2, **characterised in that** the extension shaft (7) is impinged on in the distal direction by a second spring (16).

4. The handle according to any one of claims 1 to 3, **characterised in that** the uptake mechanism (8), by means of at least one strap (8a) elastically impinged upon radially towards the outside, is formed by means of at least one radially-inwards pointing retaining stud (8b), which is formed so as to be complementary to an indentation of the tool to be inserted, and by means of at least one axially fixed locking sleeve (14), which encompasses the strap (8a) on the outside, with the internal diameter of said locking sleeve (14) decreasing in the proximal direction towards the handle (1, 1a), wherein the strap (8a) is situated in the outermost distal position of the extension shaft (7) in the region of the larger diameter (14a) or distally outside the locking sleeve (14), and the strap (8a) is situated in all the proximally following positions of the extension shaft (7) in the region of the smaller diameter of the locking sleeve (14).

5. The handle according to claim 4, **characterised in that** a blocking element (15) can be slid in the axial direction distally between the straps (8a) of the uptake mechanism (8), preferably by means of impingement by a spring element, and by the distal end (2c) of the tool (2) in the proximal direction can be pressed from the region of the straps (8a).

6. The handle according to any one of claims 1 to 5, **characterised in that** the internal diameter of the closing sleeve (11, 11a) and/or of the locking sleeve (14) changes in the form of a conical chamfer.

7. A medical tool system with a handle (1, 1a) that is connectable with a tool (2) each from a set of tools of different designs of the distal end (2a) but identical dimensions and design of the adjoining shaft (2b) and of the proximal end (2c), wherein the proximal end (2c) of the tool is undoably clamped in an uptake mechanism (8) in the handle (1, 1a) and by means of a rotational drive (4) that contains the uptake mechanism (8) can be made to rotate, and wherein the handle (1, 1a) comprises a distal guide sleeve (9, 9a) for the shaft (2b) of the tool (2), and wherein the uptake mechanism comprises a telescoping mechanism that allows axial variation of the relative position of the tool (2) relative to the guide sleeve (9, 9a) from a short position to a long position, **characterised in that** the handle is designed according to any one of claims 1 to 6.

8. The tool system according to claim 7, **characterised in that** the system comprises a set of handles (1, 1a) of different lengths of the guide sleeve (9, 9a), wherein the position of the uptake mechanism (8) for the tool (2) in its short or in its long position relative to the distal end (9b) of the guide sleeve (9, 9a) for each handle (1, 1a) is situated on the same short or long position.

9. The tool system according to claim 8, **characterised in that** in the telescoping mechanism the position of the uptake mechanism (8) for the tool (2) relative to the distal end (9b) of the guide sleeve (9, 9a) by means of a manually activateable adjustment mechanism (6) is axially adjustable in several discrete steps between the short position and the long position.

10. The tool system according to claim 9, **characterised in that** the adjustment mechanism (6) for each handle (1, 1a) relative to the external housing (3) of the handle (1, 1a) is provided in the same position, and **in that** distally to it an extension shaft (7), preferably of different length, with the uptake mechanism (8) for the tool follows.

## Revendications

1. Poignée (1a, 1a) pour système d'outil médical, comportant un boîtier (3), une commande rotative (4) à une position fixée axialement dans le boîtier (3), un mécanisme récepteur verrouillable et déverrouillable de préférence manuellement (8) pour un outil médical (2) pouvant être entraîné par la commande rotative (4) et insérable dans la poignée (1, 1a), de même qu'un manchon de guidage (9, 9a) sortant distalement de la poignée (1, 1a) et fixé axialement pour l'outil (2) et qu'un mécanisme télescopique réglable actionnable manuellement dont le mécanisme de réglage (6) actionnable manuellement est constitué par une pluralité d'entailles (7c, 7d) espacées axialement dans l'extrémité proximale (7b) de l'arbre de prolongement (7), par au moins une bille de retenue (10) fixée axialement dans le boîtier (3) mais maintenue mobile radialement et un manchon de fermeture (11, 11a) sollicité axialement par un ressort (12), comprenant les billes de retenue (10) et actionnable manuellement, dont le diamètre intérieur varie dans le sens axial, un plus petit diamètre enfonçant la ou chaque bille de retenue (10) respectivement dans une entaille (7c, 7d) de l'arbre de prolongement (7) et un plus grand diamètre dégageant la ou chaque bille de retenue (10) de l'entaille respective (7c, 7d), **caractérisée en ce que** l'entaille proximale (7d) la plus éloignée au niveau de l'arbre de prolongement (7) se trouve radialement plus éloignée vers l'extérieur pour la position courte que toutes les entailles (7c) se raccordant distalement pour la position plus longue ou longue.

2. Poignée selon la revendication 1, **caractérisée en ce que** la paroi intérieure du manchon de fermeture (11, 11a) coopérant avec la ou chaque bille de retenue (10) a une forme conique du moins au niveau (11b) du plus grand diamètre intérieur, l'angle du cône au niveau (11b) du plus grand diamètre intérieur étant supérieur à ce qu'il est au niveau (11c) du plus petit diamètre intérieur.

3. Poignée selon une des revendications 1 ou 2, **caractérisée en ce que** l'arbre de prolongement (7) est sollicité par un second ressort (16) dans le sens distal.

4. Poignée selon une des revendications 1 à 3, **caractérisée en ce que** le mécanisme récepteur (8) est constitué par au moins une bride (8a) sollicitée élastiquement radialement vers l'extérieur et comportant au moins un téton de retenue (8b) dirigé radialement vers l'intérieur et complémentaire à une entaille de l'outil à insérer et par au moins un manchon de verrouillage (14) entourant la bride (8a) par l'extérieur et fixé axialement, dont le diamètre intérieur se réduit dans le sens proximal en direction de la poignée (1, 1a), la bride (8a) se trouvant, dans la position radiale la plus à l'extérieur de l'arbre de prolongement (7), au niveau du plus grand diamètre (14a) ou distalement à l'extérieur du manchon de verrouillage (14) et la bride (8a) se trouvant, dans toutes les positions se raccordant au niveau proximal de l'arbre de prolongement (7), au niveau du diamètre plus petit du manchon de verrouillage (14).

5. Poignée selon la revendication 4, **caractérisée en ce qu'**un élément de blocage (15) peut être glissé dans le sens axial vers le sens distal entre les brides (8a) du mécanisme récepteur (8), de préférence par sollicitation au moyen d'un élément à ressort et peut être enfoncé par l'extrémité distale (2c) de l'outil (2) en direction proximale depuis la zone des brides (8a).

6. Poignée selon une des revendications 1 à 5, **caractérisée en ce que** le diamètre intérieur du manchon de fermeture (11, 11a) et/ou du manchon de verrouillage (14) varie en prenant la forme d'un biais conique.

7. Système d'outil médical comportant une poignée (1, 1a) qui peut être couplée à un outil (2) d'un jeu d'outils de différents modèles de l'extrémité distale (2a) mais de dimensionnement et modèle identique de l'arbre (2b) s'y raccordant et de l'extrémité proximale (2c), l'extrémité proximale (2c) de l'outil étant serrée de manière réversible dans un mécanisme récepteur (8) de la poignée (1, 1a) et pouvant être mise en rotation au moyen d'une commande rotative (4) contenant le mécanisme récepteur (8) et la poignée (1, 1a) présentant un manchon de guidage distal (9, 9a) pour l'arbre (2b) de l'outil (2) et le mécanisme récepteur étant pourvu d'un mécanisme télescopique qui permet une variation axiale de la position relative de l'outil (2) par rapport au manchon de guidage (9, 9a) depuis une position courte jusqu'à une position longue, **caractérisé en ce que** la poignée est réalisée selon une des revendications 1 à 6.

8. Système d'outil selon la revendication 7, **caractérisé en ce que** le système comprend un jeu de poignées (1, 1a) avec une longueur différente du manchon de guidage (9, 9a), la position du mécanisme récepteur (8) pour l'outil (2) dans sa position courte ou longue par rapport à l'extrémité distale (9b) du manchon de guidage (9, 9) pour chaque poignée (1, la) se trouvant au niveau de la même position courte ou longue.

9. Système d'outil selon la revendication 8, **caractérisé en ce que**, avec le mécanisme télescopique, la position du mécanisme récepteur (8) pour l'outil (2) par rapport à l'extrémité distale (9b) du manchon de guidage (9, 9a) est réglable axialement au moyen d'un mécanisme de réglage actionnable manuellement (6) à plusieurs étapes distinctes entre la position courte et la position longue.

10. Système d'outil selon la revendication 9, **caractérisé en ce que** le mécanisme de réglage (6) pour chaque poignée (1, 1a) est prévu à la même position par rapport au boîtier extérieur (3) de la poignée (1, 1a) et qu'un arbre de prolongement (7) de préférence de longueur respectivement différente se raccorde distalement au mécanisme récepteur (8) pour l'outil (2).
